# EUROPEAN PATENT APPLICATION

(11) **EP 2 444 404 A1**
(43) Date of publication of application: **25.04.2012**
(21) Application number: 10306093.5
(22) Date of filing: 07.10.2010
(51) Int. Cl.: C07F 7/00, C07F 9/90, C07D 345/00, C23C 30/00

(54) **Metal compounds for deposition of chalcogenide films at low temperature**

(71) Applicant: L'Air Liquide Société Anonyme pour l'Etude et l'Exploitation des Procédés Georges Claude, 75007 Paris (FR)
(72) Inventor: Blasco, Nicolas, 38000, GRENOBLE (FR); Gatineau, Julien, IBARAKI Ibaraki 300-0848 (JP); Girard, Jean-Marc, TOKYO 162-0062 (JP); Lahootun, Vanina, 91270, VIGNEUX SUR SEINE (FR); Okubo, Shingo, Tsukuba-shi Ibaraki 305-0821 (JP); Zauner, Andreas, 78960, VOISINS LE BRETONNEUX (FR)
(74) Representative: Grout de Beaufort, François-Xavier

(57) **Abstract**

Compounds of formula: 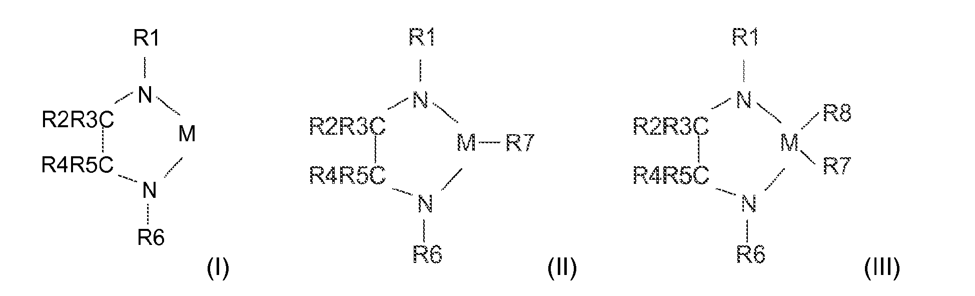 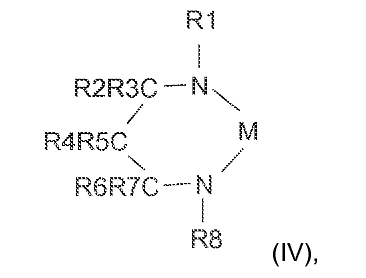 wherein M is selected from Ge, Te, and Sb and each R is independently selected from H, C1-C6 alkyl, C1-C6 alkylsilyl, C1-C6 perfluorocarbon, dialkylamino NR'R" (R', R" are independently selected from H, C1-C6 alkyl, C1-C6 alkylsilyl, C1-C6 perfluorocarbon), cyano, carbonyl, phenyl, benzyl, pyridyl, halogens such as F, Cl, Br, and I, sulfo, nitro, carboxy, hydroxyl, diazo, azido.

## Description

Recently, requirements of metalorganic compounds as chemical vapor deposition (CVD) and/or atomic layer deposition (ALD) precursors for semiconductor manufacturing process are increasing because of the higher surface coverage of deposited films than that obtained by physical vapor deposition method (PVD, sputtering method). For example, production of GeSbTe alloy thin films, which are used in phase change memory (PCM) device, is changing from PVD to CVD/ALD so as to increase the capacity of memory cells.

There are many kinds of CVD/ALD precursor materials, be it commercially available compounds or not, but they are roughly categorized into few types according to the element bonding to metal atom(s), M-H, M-C, M-N, M-O, M-X (X: halogens), and others. Especially alkylamino metal compounds, M(NRR')n (n depends on the valence state of the metal), are one of the most useful metalorganic precursors because of preferable thermal stability, high reactivity, low carbon contamination of deposited films, and availability of the ALD condition. Some publications about metal amidinate compounds (amidinate formula: (R₁)NC(R₂)N(R₃)) as CVD/ALD precursors have been submitted. Several research studies on several (from 2 to 4) element containing layers (such as GeSbTe, GeSbInTe, GaLaS...) of chalcogenide materials in PCM applications have been carried out. Especially, Ge₂Sb₂Te₅ films are widely studied because of the material's low melting temperature which is desired to decrease the thermal budget during usage. A constant and exact value of the ratio of elements inside such chalcogenide materials is required in order to control the film properties.

Phase Change Memory (PCM) refers to a novel memory technology based on chalcogenide materials that undergo a phase change via a heater and are read out as "0" or "1" based on their electrical resistivity, which changes in correspondence to whether the phase change material in the cell is in the crystalline or amorphous phase.

The chalcogenide materials used in PCM comprise a large number of binary, ternary, and quaternary alloys of a number of metals and metalloids. Examples include GeSbTe, GeSbInTe, and many others.

PCM devices require relatively pure chalconide material alloys, with well controlled composition. Current processes for making PCM devices utilize physical vapor deposition to deposit thin films of these chalconide materials. The thick planar structures of the current generation are well-served by PVD.

As device geometries shrink, the chalconide material must be deposited into vias in order to control the phase transition and the necessary heat transfer. Such implementation of chalconide materials can also be beneficial in improving reliability of small volume devices. A major deficiency in the current art is the requirement of high deposition temperatures needed for conventionally employed alkyl (e.g., Me₃Sb, Me₂Te) or halide sources. These temperatures are typically well in excess of 300 degrees centigrade, and may for example be on the order of 500 degrees centigrade. Such high temperatures substantially exceed the thermal budget for device integration and can result in the evaporation of the chalcogenide, rendering the product PCM device deficient or even useless for its intended purpose.

The art continues to seek improvements in the art of PCM devices, including improvements in manufacturing techniques and improved precursors useful for forming memory device structures.

The physical vapor deposition (PVD, sputtering method) is used to deposit the alloy in M-bit size samples now, but the use of PVD should be avoided because of the inherent difficulties to deposit uniform films in deep trenches that are present in the G-bit size memories. For such small patterns, chemical vapor deposition (CVD) and/or atomic layer deposition (ALD) are essential techniques to make small sized PCM devices.

Few CVD (ALD) methods are reported in prior arts such as WO2008008319, WO2003083167, WO002027063, WO 2007/133837 or WO 2008/057616.

It is known that the need of thermal processes for the deposition of chalcogenide films, or any other metal based films (metallic, nitride, oxide, ...) is very important, as until now mainly plasma processes were developed. This is because the current precursors are not reactive enough to allow deposition in thermal ALD mode, and/or their respective deposition window does not fit with other precursors. The deposition of chalcogenide films itself in thermal ALD process is also desired, in order to answer to the downsizing of the devices and the introduction of sever architecture (high aspect ratio, 3D) devices. Besides, deposition at low temperature is also desired to limit the thermal budget during the deposition process, as well as to limit issues as the melting point of chalcogenide materials is low.

This is why the present invention concerns the compounds of formula: 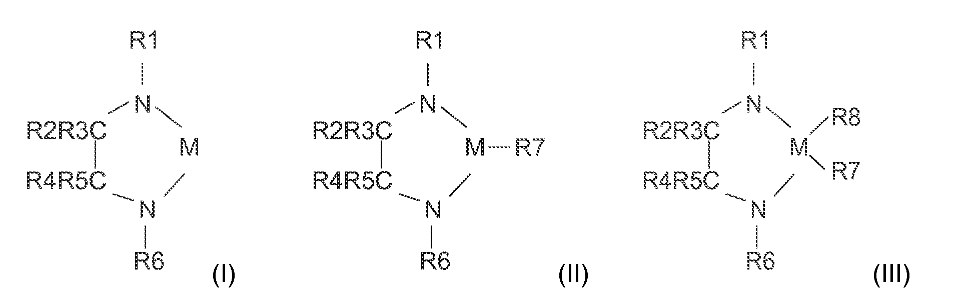 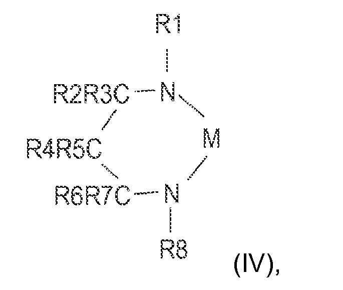 wherein M is selected from Ge, Te, and Sb and each R is independently selected from H, C1-C6 alkyl, C1-C6 alkylsilyl, C1-C6 perfluorocarbon, dialkylamino NR'R" (R', R" are independently selected from H, C1-C6 alkyl, C1-C6 alkylsilyl, C1-C6 perfluorocarbon), cyano, carbonyl, phenyl, benzyl, pyridyl, halogens such as F, Cl, Br, and I, sulfo, nitro, carboxy, hydroxyl, diazo, azido.

According to other embodiments, the present invention concerns:
- Compounds selected from:
   Ge(CH₃NCH₂CH₂NCH₃),
   Ge(C₂H₅NCH₂CH₂NC₂H₅),
   Ge((C(CH₃)₃NCH₂CH₂NC(CH₃)₃),
   Ge(CH₃NC(CH₃)HC(CH₃)HNCH₃),
   Te(CH₃NCH₂CH₂NCH₃),
   Te(C₂H₅NCH₂CH₂NC₂H₅),
   Te((C(CH₃)₃NCH₂CH₂NC(CH₃)₃),
   Te(CH₃NC(CH₃)HC(CH₃)HNCH₃).
- Compounds selected from:
   Sb(N(CH₃)CH₂CH₂N(CH₃))H,
   Sb(N(CH₃)CH₂CH₂N(CH₃))(NMe₂),
   Sb(N(CH₃)CH₂CH₂N(CH₃))(CH₃),
   Sb(N(CH₃)CH₂CH₂N(CH₃))(C₂H₅),
   Sb(NC(CH₃)₃CH₂CH₂NC(CH₃)₃)(NMe₂),
   Sb(NC(CH₃)₃CH₂CH₂NC(CH₃)₃)(CH₃),
   Sb(NC(CH₃)₃CH₂CH₂NC(CH₃)₃)(C₂H₅).
- Compounds selected from:
   Ge(N(C(CH₃)₃)CH₂CH₂N(C(CH₃)₃)H₂,
   Ge(N(C(CH₃)₃)CH₂CH₂N(C(CH₃)₃)(N(CH₃)₂)₂,
   Ge(N(C(CH₃)₃)CH₂CH₂N(C(CH₃)₃)(CH₃)₂,
   Ge(N(C(CH₃)₃)CH₂CH₂N(C(CH₃)₃)(C₂H₅)₂,
   Ge(N(C(CH₃)₃)CH₂CH₂N(C(CH₃)₃)C1₂.
- Compounds selected from:
   Ge(N(CH₃)CH₂CH₂CH₂N(CH₃)),
   Ge(NC(CH₃)₃CH₂CH₂CH₂N(CH₃)₃).

According to another embodiment, the invention concerns a method of forming a germanium (Ge) - antimony (Sb) - tellurium (Te) (GST) phase change memory material on a substrate, comprising : a) contacting the substrate with at least one precursor as defined above for a phase change memory chalcogenide alloy under conditions; b) producing deposition of the chalcogenide alloy on the substrate, wherein said conditions comprise temperature below 250 degrees centigrade and said contacting step a) comprises chemical vapor deposition or atomic layer deposition.

According to other embodiments, the present invention concerns:
- A method as defined above, wherein the chalcogenide material is of the formula GeₓSb_{y}Te_{z} (GST), in which x, y, z are comprised between 0 and 1.
- A method as defined above, wherein the deposition process is performed in a temperature range of 25°C to 250°C, preferably in the range of 25°C to 200°C, and more preferably in the range of 25°C to 150°C.
- A method as defined above, wherein the film is deposited in thermal mode or plasma mode by Chemical Vapor Deposition (CVD) or Atomic Layer Deposition (ALD) process, or equivalent techniques like Plasma-enhanced CVD (PE-CVD) and plasma-enhanced ALD (PE-ALD).
- A method as defined above, wherein the film is deposited in thermal Atomic Layer Deposition (ALD) process.
- A method as defined above, wherein the substrate is selected among metal or metal nitride films such as tungsten, titanium nitride, titanium aluminum nitride.
- A method as defined above, wherein a co-reactant selected among hydrogen, ammonia, hydrazine, diethylsilane, and mixtures thereof is introduced to improve the deposition process.
- A method as defined above, wherein at least a doping element can be added to the GST film, said doping element is selected among silicon, nitrogen, oxygen.

Lower temperatures processes can be obtained when the precursor(s) is mixed with other adequately selected metalorganic or inorganic molecules.

The present invention relates to systems and processes for deposition of phase change memory material on substrates, for fabrication of a phase change memory devices. The invention relates in one aspect to a method of forming a phase change memory material on a substrate, comprising contacting the substrate with precursors for a phase change memory chalcogenide alloy under conditions producing deposition of the chalcogenide alloy on the substrate, wherein such conditions comprise temperature below 250 degrees centigrade and such contacting comprises chemical vapor deposition or atomic layer deposition.

In another aspect, the invention relates to a method of forming a germanium-antimony-tellurium phase change memory material on a substrate, comprising contacting the substrate with precursors for a phase change memory germanium-antimony-tellurium alloy under conditions producing deposition of the germanium-antimony-tellurium alloy on the substrate, wherein such conditions comprise temperature below 250 degrees centigrade and such contacting comprises chemical vapor deposition or atomic layer deposition, with the precursors comprising at least one halide precursor.

Additional aspects of the invention relate to PCM films formed in accordance with the present invention; corresponding devices; tellurium complexes, germanium complexes, germanium tellurides, and processes utilizing same for forming GST films; compositions including combinations of precursors for forming PCM films; and packaged precursors adapted for coupling to a deposition tool comprising such compositions. More specifically, the invention in one aspect relates to chalcogenide alloys, and to their low temperature deposition e.g., by chemical vapor deposition (CVD) or atomic layer deposition (ALD), to form PCM devices. CVD and ALD methods are employed in the practice of the present invention to achieve scalability to large area wafers and for composition control.

Preferred chalconide alloys include alloys including two or more of germanium, antimony and tellurium.

As used herein, the term "low temperature" means a temperature below 250 degrees centigrade

The temperature at which the PCM material is deposited is preferably less than 200 degrees centigrade and most preferably less than 150 degrees centigrade.

The advantages of chemical vapor deposition and atomic layer deposition at low deposition temperature in the fabrication of PCM devices include substantial improvement of read/re-write times in small devices, as a result of the high conformality of the deposited PCM material.

Such method may further include fabricating said phase change memory material into a phase change memory device.

### Synthesis of the molecules

### Example 1: synthesis of dichloro (N,N'-bis-tert-butyl-1,4-diazabutadiane) germanium [1].

Dichloro(N,N'-bisterbutyl-1,4-diazabutadiane) germanium was obtained by reacting a solution of GeCl₄ and the corresponding diamine in presence of triethylamine. [1] is obtained in quantitative yields.

### Example 2: synthesis of Bis(dimethylamino) (N,N'-bis-tert-butyl-1,4-diazabutadiane) germanium [2].

[1] was reacted with lithiumdimethylamide to obtain the targeted product, after purification by sublimation. The molecule is a colourless liquid at 25°C.

### Example 3: synthesis of (N,N'-bisterbutyl-1,4-diazabutadiane) germanium [3].

The divalent (N,N'-bis-tert-butyl-1,4-diazabutadiane) germanium was obtained in a similar way than Bis(dimethylamino)(N,N'-bis-tert-butyl-1,4-diazabutadiane) germanium. However, a reductive dehalogenation was performed on [1] in THF solution with lithium granules. After a few hours, the solvent was removed under vacuum and replaced with n-pentane followed by filtration and evaporation of the solvent. Potential impurities can be removed by sublimation, and the synthesized product is a colourless solid (MP: 45 °C, quantitative yield).

Other synthesis routes were also evaluated using GeCl₂ and dioxane, leading to similar results.

### Example 4: synthesis of diethyl (N,N'-bis-tert-butyl-1,4-diazabutadiane) germanium [4].

Diethyl (N,N'-bis-tert-butyl-1,4-diazabutadiane) germanium was obtained by reacting [1] with MeLi, followed by the purification steps mentioned in example 2. The molecule is a colourless liquid.

### Thermal characterization of the molecules

All the thermo-gravimetric analyses were performed in an inert atmosphere in order to avoid reaction of the molecules with air and moisture (same atmosphere encountered in the deposition process).

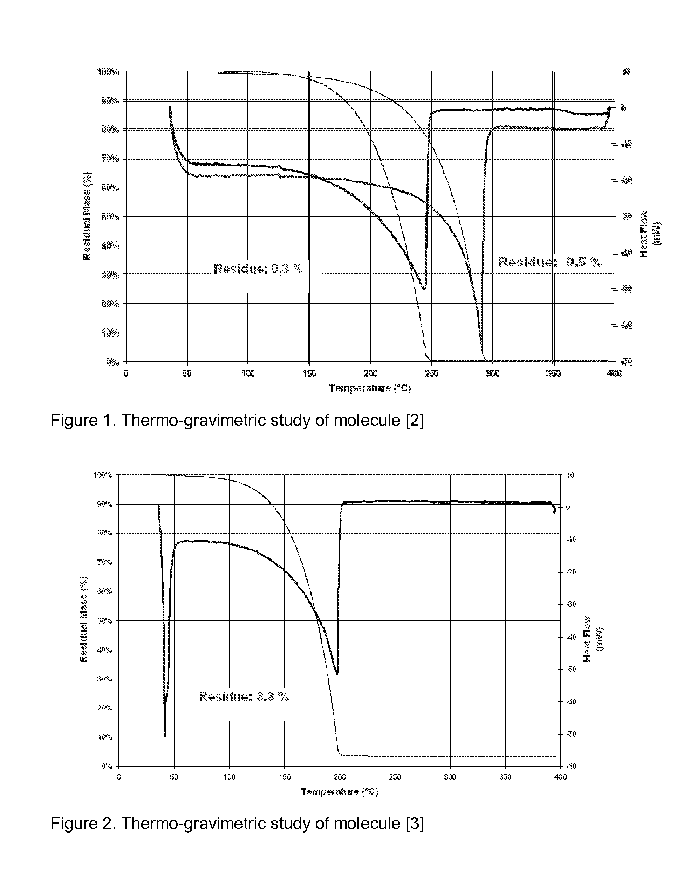

The thermal stability and volatility of molecule [2] was studied by thermogravimetry analysis, as shown in figure 1. The colourless liquid is cleanly vaporized without leaving residues, advocating for a good stability below 300°C (no residues in closed-cup conditions), which is far above the deposition temperature and delivery temperature. The vapor pressure of this molecule was evaluated around 270 Pa at 100°C, which is enough for deposition process (given its high thermal stability). This molecule is thus very relevant for deposition process of germanium-based films (GST for instance).

Thermal stability of [3] is showed in figure 2. The vaporization of the molecule was smooth until 200°C, where it stops leaving a very few amount of residuals (3.3%). The final vaporization temperature is 200°C, much below the 250°C obtained with molecule [2], due to the higher volatility of [3].

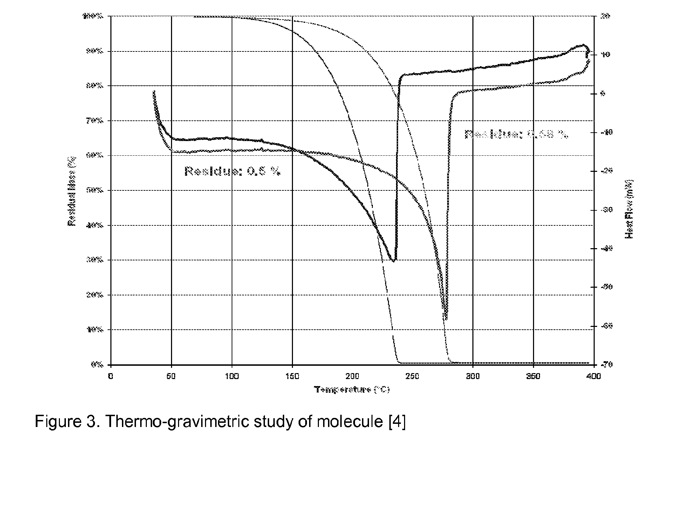

Figure 3 shows the thermo-gravimetric results of molecule [4]. This liquid molecule is also very stable, as proven by the extremely low residual amount obtained, even in closed cup case (0.58%). The volatility of the molecule seems to be between [2] and [3], as revealed by the final vaporization temperature. This molecule also presents all the properties required for a deposition process.

### Deposition tests

### Example 5: germanium films deposited using bis(dimethylamino) (N,N'-bis-tert-butyl-1,4-diazabutadiane) germanium.

Germanium films were deposited from temperatures as low as 200°C. Vapors of [2] were delivered to a reaction furnace using N₂ as a carrier gas, as well as for dilution purpose. Tests were done with hydrogen as reactant.

Germanium films were obtained in ALD mode at temperatures below 250°C. The concentrations of various elements into the deposited metal films were analyzed by an Auger spectrometry and the values for all elements (carbon, nitrogen, oxygen) were below the detection limit of the apparatus.

### Example 6: GeSbTe (GST) films deposited using bis(dimethylamino) (N,N'-bis-tert-butyl-1,4-diazabutadiane) germanium [2].

In the same way as example 4, molecule [2] was used together with tellurium (Te) and antimony (Sb) molecules to make GST films in thermal mode. Bis(isopropoxide) tellurium, bis(triethylsilyl)tellurium, bis(dimethyl-tert-butylsilyl) tellurium, bis(bis(trimethylsilylamino)) tellurium, (N,N'-bis-tert-butyl-1,4-diazabutadiane) tellurium and tris(dimethylamino) antimony, tris(trimethylsilyl) antimony, and dimethylamino (N,N'-bis-tert-butyl-1,4-diazabutadiane) antimony were among the tested Te and Sb precursors, respectively.

In Atomic Layer Deposition (ALD) mode, it was proved that the use of molecule [2] enables to decrease the deposition temperature of the GST films compared with a process that uses other germanium molecules, such as tetrakis(dimethylamino) germanium (TDMAG). In the same deposition conditions, the deposition temperature could be lowered by about 50°C by using molecule [1] (vs. TDMAG). When an alkylsilyl tellurium molecule or an alkylsilyl antimony molecule was used together with molecule [2], the deposition temperature could be decreased even further.
It was remarked during the evaluation process that the deposition temperature could be lowered more drastically using [1] and the alkylsilyl tellurium and alkylsilyl antimony precursors. In such case, depositions were obtained at temperatures as low as 100°C. Auger analyses enables to verify that the level of carbon in the obtained films was below the detection limit (∼1%), which proved the superior quality of the films.

The use of metal-heterocyclic compounds of the present invention as CVD/ALD precursors for deposition of chalcogenide films has advantages relative to corresponding amidinate precursors in terms of the process temperature and impurity content. It will also allow the deposition of films by thermal processes (CVD, ALD, pulse CVD), and not only by plasma enhanced type of technology.

## Claims

1. Compounds of formula: 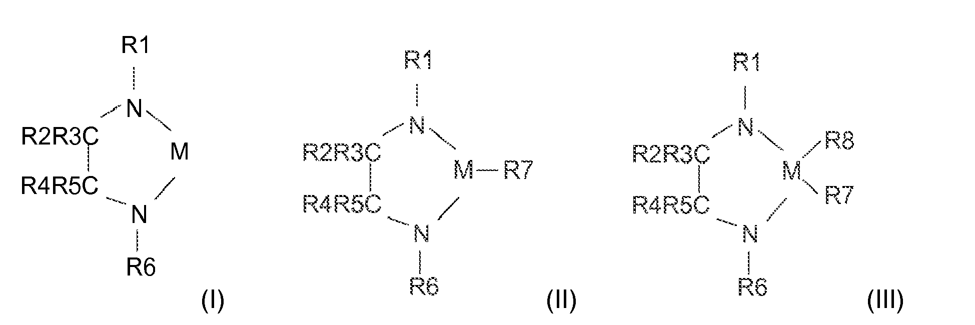 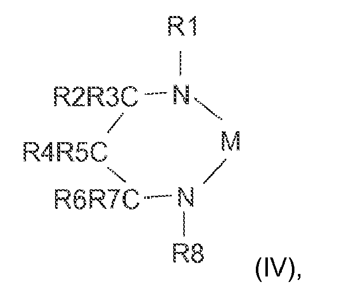 wherein M is selected from Ge, Te, and Sb and each R is independently selected from H, C1-C6 alkyl, C1-C6 alkylsilyl, C1-C6 perfluorocarbon, dialkylamino NR'R" (R', R" are independently selected from H, C1-C6 alkyl, C1-C6 alkylsilyl, C1-C6 perfluorocarbon), cyano, carbonyl, phenyl, benzyl, pyridyl, halogens such as F, Cl, Br, and I, sulfo, nitro, carboxy, hydroxyl, diazo, azido.

2. Compounds of claim 1 selected from:
Ge(CH₃NCH₂CH₂NCH₃),
Ge(C₂H₅NCH₂CH₂NC₂H₅),
Ge((C(CH₃)₃NCH₂CH₂NC(CH₃)₃),
Ge(CH₃NC(CH₃)HC(CH₃)HNCH₃),
Te(CH₃NCH₂CH₂NCH₃),
Te(C₂H₅NCH₂CH₂NC₂H₅),
Te((C(CH₃)₃NCH₂CH₂NC(CH₃)₃),
Te(CH₃NC(CH₃)HC(CH₃)HNCH₃).

3. Compounds of claim 1 selected from:
Sb(N(CH₃)CH₂CH₂N(CH₃))H,
Sb(N(CH₃)CH₂CH₂N(CH₃))(NMe₂),
Sb(N(CH₃)CH₂CH₂N(CH₃))(CH₃),
Sb(N(CH₃)CH₂CH₂N(CH₃))(C₂H₅),
Sb(NC(CH₃)₃CH₂CH₂NC(CH₃)₃)(NMe₂),
Sb(NC(CH₃)₃CH₂CH₂NC(CH₃)₃)(CH₃),
Sb(NC(CH₃)₃CH₂CH₂NC(CH₃)₃)(C₂H₅).

4. Compounds of claim 1 selected from:
Ge(N(C(CH₃)₃)CH₂CH₂N(C(CH₃)₃)H₂,
Ge(N(C(CH₃)₃)CH₂CH₂N(C(CH₃)₃)(N(CH₃)₂)₂,
Ge(N(C(CH₃)₃)CH₂CH₂N(C(CH₃)₃)(CH₃)₂,
Ge(N(C(CH₃)₃)CH₂CH₂N(C(CH₃)₃)(C₂H₅)₂,
Ge(N(C(CH₃)₃)CH₂CH₂N(C(CH₃)₃)Cl₂.

5. Compounds of claim 1 selected from:
Ge(N(CH₃)CH₂CH₂CH₂N(CH₃)),
Ge(NC(CH₃)₃CH₂CH₂CH₂N(CH₃)₃).

6. A method of forming a germanium (Ge) - antimony (Sb) - tellurium (Te) (GST) phase change memory material on a substrate, comprising : a) contacting the substrate with at least one precursor of any one of claim 1 to 5 for a phase change memory chalcogenide alloy under conditions; b) producing deposition of the chalcogenide alloy on the substrate, wherein said conditions comprise temperature below 250 degrees centigrade and said contacting step a) comprises chemical vapor deposition or atomic layer deposition.

7. Method according to claim 6, wherein the chalcogenide material is of the formula GeₓSb_{y}Te_{z} (GST), in which x, y, z are comprised between 0 and 1.

8. Method of claim 6 or 7, wherein the deposition process is performed in a temperature range of 25°C to 250°C, preferably in the range of 25°C to 200°C, and more preferably in the range of 25°C to 150°C.

9. Method according to claim 8, wherein the film is deposited in thermal mode or plasma mode by Chemical Vapor Deposition (CVD) or Atomic Layer Deposition (ALD) process, or equivalent techniques like Plasma-enhanced CVD (PE-CVD) and plasma-enhanced ALD (PE-ALD).

10. Method of claim 9, wherein the film is deposited in thermal Atomic Layer Deposition (ALD) process.

11. Method according to anyone of claim 6 to 10, wherein the substrate is selected among metal or metal nitride films such as tungsten, titanium nitride, titanium aluminum nitride.

12. Method according to anyone of claim 6 to 11, wherein a co-reactant selected among hydrogen, ammonia, hydrazine, diethylsilane, and mixtures thereof is introduced to improve the deposition process.

13. Method according to one of claim 6 to 12, wherein at least a doping element can be added to the GST film, said doping element is selected among silicon, nitrogen, oxygen.
